(19) Europäisches Patentamt

European Patent Office

Office européen des brevets

(11) Veröffentlichungsnummer: **0 214 620 B1**

(12) **EUROPÄISCHE PATENTSCHRIFT**

(45) Veröffentlichungstag der Patentschrift: **29.04.92**

(21) Anmeldenummer: **86112243.0**

(22) Anmeldetag: **04.09.86**

(51) Int. Cl.⁵: **A61K 47/00**, A61K 31/55, A61L 15/16

(54) Transdermale Applikationsform von Diltiazem.

(30) Priorität: **04.09.85 DE 3531545**

(43) Veröffentlichungstag der Anmeldung:
**18.03.87 Patentblatt 87/12**

(45) Bekanntmachung des Hinweises auf die
Patenterteilung:
**29.04.92 Patentblatt 92/18**

(84) Benannte Vertragsstaaten:
**AT CH DE FR GB IT LI**

(56) Entgegenhaltungen:
**EP-A- 0 152 281**
**EP-A- 0 189 861**

**PATENT ABSTRACTS OF JAPAN, Band 9, Nr. 28, (C-264) (1751), 6 Februar 1985 & JP-A-59 175415**

**JOURNAL OF PHARMACEUTICAL SCIENCES, Band 73, Nr. 8, August 1984, Seiten 1153-1156; Am. Pharm. Ass., Washington, US**

**CHEMICAL ABSTRACTS, Band 96, Nr. 6, 8 Februar 1982, Seite 378, Ref. Nr. 40902j; Columbus, Ohio, US**

(73) Patentinhaber: **GÖDECKE AKTIENGESELL-
SCHAFT
Salzufer 16
W-1000 Berlin 10(DE)**

(72) Erfinder: **Bauer, Kurt-Heinz, Prof. Dr.
Im Finkeler 4
W-7800 Freiburg 33(DE)**
Erfinder: **Ouade, Dittmar
Sonnhalde 66A
W-7803 Gundelfingen(DE)**
Erfinder: **Mahjour, Majid
50-3B Mt. Pleasant Village
Morris Plains N.J. 07950(US)**

**Beschreibung**

Calciumantagonisten stellen wichtige Wirkstoffe für die Vorbeugung und Behandlung coronarer Erkrankungen und peripherer Durchblutungsstörungen dar. Aufgrund ihres relativ hohen First Pass Effekts ist jedoch bei peroraler Verabreichung nur ein relativ geringer Wirkstoffanteil wirksam, d.h. es müssen der Bioverfügbarkeit entsprechend höhere Dosen vorgesehen werden. Die perorale Bioverfügbarkeit von beispielsweise Diltiazem liegt bei 40 - 60 %. Eine parenterale Verabreichung kann in der Regel nur von entsprechend geschultem Personal durchgeführt werden, so daß die Selbstmedikation durch den Patienten ausscheidet. Bei gewissen Wirkstoffen, nämlich solchen, die eine sehr geringe Molekülgröße aufweisen, eine polare Struktur besitzen und im Bereich unterhalb von 5 mg/Dosis wirksam sind, konnten bereits transdermale Applikationsformen verwirklicht werden. So wurden in jüngerer Zeit sogenannte Nitrat-Pflaster zur Behandlung von Angina Pectoris entwickelt, bei denen eine ausreichende Wirkstoffmenge von ca 1 mg/Dosierung/Tag Nitroglycerin in der Lage ist, die Haut zu penetrieren. Ähnlich geringe Dosierungen können bei dem potenten Antihypertonicum und Diureticum Clonidin wirksam transdermal appliziert werden.

Calciumantagonisten erfordern, abgesehen vom First Pass Effekt, jedoch in der Regel vergleichsweise hohe Dosierungen (Faktor 10 - 100) und sind bereits von ihrer Molekülgröße her mit Nitroglycerin oder Clonidin nicht zu vergleichen. Sie sind auch von der Struktur her allesamt nur wenig polar und in gewissem Sinn vorwiegend symmetrisch aufgebaut.

Gleichwohl wurde überraschend gefunden, daß Diltiazem-Base in einem hautverträglichen Lösungsmittel gelöst, in der Lage ist, bei perkutaner Applikation ausreichende Blutspiegel zu bilden und über einen Zeitraum von 24 Stunden aufrecht zu erhalten.

Gegenstand der Erfindung ist daher eine transdermale Applikationsform für Menschen und Säugetiere, enthaltend Diltiazem-Base als Wirkstoff der, gegebenenfalls mit an sich bekannten Hilfs- und Zusatzstoffen, in einem hautverträglichen Lösungsmittel gelöst oder suspendiert ist.

Solche perkutane Applikationsformen haben den großen Vorteil, daß, anders als bei peroralen Applikationsformen oder etwa bei Injektionsformen über lange Zeit gleichmäßige Blutspiegel ohne Schwankungen aufrecht erhalten werden können. Darüber hinaus tritt, wie erwähnt, bei percutaner Applikation kein First Pass Effekt auf. Die Applikation etwa in Form eines entsprechend imprägnierten Pflasters ist außerdem sehr einfach und muß nur einmal am Tag vorgenommen werden.

Die Herstellung entsprechender transdermaler Applikationsformen kann in analoger Weise, wie bei den bekannten Nitratpflastern, etwa durch Tränken von entsprechend bemessenen (10 - 50 cm$^2$, bevorzugt ca. 25 - 35 cm$^3$) Pflastern mit einer Lösung des Wirkstoffs, die vorzugsweise zusätzlich spreitende oder gut netzende Hilfsstoffe, wie Isopropylmyristat, PEG 6-Caprinsäure, Caprylsäure-Glycerid oder Ethyloleat enthalten sollten. Da Salzformen bekanntlich die Haut kaum durchdringen können.

Die orale Tagesdosis von Diltiazem beträgt 180 mg. Es ist als äußerst überraschend zu bezeichnen, daß bei transdermaler Applikation gleichwertige Blutspiegel erzielt werden können.

Die Wirksamkeit der neuen Applikationsform wurde bisher an haarlosen Mäusen sowie am Schwein getestet. Bei beiden Tierarten wurde die Wirksamkeit bestätigt. Die Wirksamkeit am Schwein ermöglicht über den Einsatz im Humanbereich hinaus auch eine sehr effektive Verwendung im Veterinärbereich.

Es ist nämlich bekannt, daß Zuchtschweine unter hoher Streßbelastung stehen, wenn sie transportiert oder in eine für sie fremde Umgebung gebracht werden. Dieser Transportstreß wirkt sich besonders negativ aus, wenn die Tiere in großer Zahl ohne den notwendigen natürlichen Freiraum oder in engen Boxen transportiert werden.

Unter extrem hoher Streßbelastung stehen auch Eber beim Deckakt und hochwertige Zuchtschweine auf landwirtschaftlichen Ausstellungen. Bisher hat man versucht, die Streßbelastung durch einen direkten Einfluß auf das zentrale Nervensystem mittels Tranquilizern zu mindern. Diese Maßnahme trägt zwar dazu bei, die hohen Ausfallquoten durch Schocktod in Grenzen zu halten, führt aber andererseits dazu, daß die Tiere schläfrig und träge werden, so daß Eber nicht die erforderliche Deckleistung bringen können und Zuchtschweine auf Ausstellungen ein entsprechend trauriges Bild bieten.

Schlachtschweine hinwiederum werden zwar durch höhere Dosen Tranquilizer beruhigt, jedoch ist es Vorschrift, die Schlachtung erst nach einem festgelegten Zeitabstand nach der Verabreichung der Medikamente durchzuführen, um sicherzustellen, daß das gewonnene Schlachtgut keine bedenklichen Gewebespiegel mehr aufweist.

All dies zeigt, daß die Anwendung von Tranquilizern bisher zwar notwendig war aber auch von einer Reihe unvermeidbarer Nachteile begleitet ist.

Die erfindungsgemäße Applikationsform gestattet es, Säugetiere allgemein vor den unerwünschten Folgen einer unumgänglichen Streßbelastung zu schützen.

Darüber hinaus ist es bisher nicht bekannt, zur Minderung der Streßbelastung bei Tieren, insbesondere Schweinen, überhaupt Calciumantagonisten einzusetzen.

Man erreicht hierdurch, daß z.B. Schweine auch unter stärkster Streßbelastung nicht mehr zusammenbrechen. Es hat sich nämlich herausgestellt, daß das Verenden der Tiere nicht auf einen Schocktod sondern in der Regel auf einen partiellen Untergang von Herzmuskelgewebe infolge der durch die Streßeinwirkung unnatürlich hohen Herzfrequenz und damit unausgeglichenen $O_2$-Bilanz zurückzuführen ist.

Es kommt also nicht darauf an, wie es bisher von der Fachwelt als selbstverständlich angenommen wurde, die nervliche Belastung der Tiere mittels beruhigender Arzneimittel zu reduzieren, sondern es ist tatsächlich so, daß die Tiere den bisher als kaum vermeidbare Gefahr angesehenen Streß mit überraschender Leichtigkeit überstehen, wenn für eine Ökonomisierung der Durchblutung des Herzmuskelgewebes Sorge getragen wird. Eine ZNS-Dämpfung erübrigt sich damit.

Ein weiterer Gegenstand der vorliegenden Erfindung ist demnach die Verwendung von Diltiazem-Base für die Herstellung von Arzneimitteln zur Bekämpfung der durch Streßbelastung entstehenden Schäden bei Nutztieren.

An sich kommt es zwar auf die Art der Applikation von Diltiazem-Base zu diesem Zweck nicht wesentlich an, wenn nur dafür gesorgt wird, daß ausreichend hohe Gewebespiegel (etwa 50 - 100 ng/ml) erzielt werden und daß die Resorption beim Tier so rasch wie möglich erfolgt.

Grundsätzlich eignet sich daher auch die Injektion (vorwiegend i.m.), und die orale Gabe, wenn man deren Nachteile in Kauf nimmt. Bei i.m. Injektionen wären etwa 0,8 - 0,9 mg Wirkstoff pro kg anzusetzen.

Für perorale Formen, die man dem Futter oder Trinkwasser zusetzen kann, müßten entsprechend höhere Dosen (ca. 1,7 - 1,9 mg/kg) angewandt werden

Bei umfangreichen Versuchen hat es sich aber für die Anwendung herausgestellt, daß sich insbesondere beim Hausschwein die erfindungsgemäße transdermale Applikationsform am Schweineohr besonders gut eignet, da sie besonders bequem anwendbar ist und reproduzierbar rasch hohe therapeutische Wirkspiegel ergibt.

Für diese Applikationsform wird der Wirkstoff Diltiazem-Base in einer neutralen Trägersubstanz gelöst oder suspendiert und in dieser Form relativ großflächig auf der Außenhaut des Schweineohrs appliziert. Überraschend stellen sich innerhalb weniger Minuten (15 - 25 Min.) bereits ausreichende Gewebespiegel ein. Noch günstiger ist es, mit der Lösung oder Suspension einen saugfähigen Träger bestimmter Oberfläche zu tränken. Als saugfähiger Träger kommt z.B. ein Mull oder Gazequadrat von 3 - 10 cm Kantenlänge infrage.

Da Diltiazem, anders als Tranquilizer, wie z.B. Chlorpromazin oder solche der Azepam-Reihe, eine sehr niedrige Halbwertszeit hat, ist eine unzulässige Gewebsbelastung des Schlachtguts durch den Wirkstoff problemlos zu vermeiden. Insbesondere werden die Wartezeiten drastisch gekürzt.

Die transdermale Applikationsform ist überdies besonders leicht variierbar. Wenn es bei Schweinen darum geht, nicht nur kurzfristig, sondern über einen längeren Zeitraum den therapeutischen Wirkspiegel auf einem gewünschten Niveau zu halten, so ist es beispielsweise möglich, eine galenische Zubereitung in einer Reservoirpackung am Ohr des Schweines zu fixieren. Der Wirkstoff kann dann über eine längere Zeit durch die Haut penetrieren und das Tier rund um die Uhr vor lebensbedrohenden Auswirkungen der Streßbelastung, z.B. Umstallung, Transport oder Deckakt schützen.

Für Schweine ist die transdermale Applikationsform daher ebenfalls besonders geeignet.

Ein weiterer Gegenstand der Erfindung ist daher eine transdermale Applikationsform für Säugetiere, enthaltend Diltiazem-Base als Wirkstoff und übliche Hilfs- und Zusatzstoffe.

Die transdermalen Applikationsformen enthalten vorzugsweise organische, gut hautverträgliche Lösungsmittel wie Ethanol, Methylpyrrolidon, Polyethylenglycol, Oleylalkohol, Octanol, Linolsäure, Triacetin, Propylenglykol, Glycerin, Solketal oder Dimethylsulfoxid, die die Penetration durch die Haut erleichtern.

Bevorzugt als Lösungsmittel werden Linolsäure und Propylenglykol verwendet, und zwar in einem Gewichtsverhältnis von 1 - 20 Gew.-% Linolsäure und 99 - 80 Gew.-% Propylenglykol, bevorzugt 10 Gew.-% Linolsäure und 90 Gew.-% Propylenglykol.

Ein weiterer Gegenstand der Erfindung ist ein Verfahren zur Herstellung einer transdermalen Applikationsform eines Wirkstoffs für Menschen und Säugetiere, bei welchem der Wirkstoff Diltiazem-Base ist, welcher in einem der genannten hautverträglichen Lösungsmittel, gegebenenfalls mit weiteren Hilfs- und/oder Zusatzstoffen, gelöst oder suspendiert wird.

Zum Nachweis der Wirksamkeit der transdermalen Zubereitungsformen wurden folgende Versuche durchgeführt:

Die Durchdringungsfähigkeit von Diltiazem-Base durch die Haut haarloser Mäuse wurde untersucht.

Aus einer Lösung von 3 g Diltiazem.Hydrochlorid in 30 ml destilliertem Wasser wurde durch tropfenweise Zugabe von Ammoniumhydroxydlösung Diltiazem-Base ausgefällt. Die Base wurde mit 3 x 20 ml Methylenchlorid extrahiert. Die Methylenchloridfraktion wurde mittels wasserfreiem Na-Sulfat getrocknet. Nach dem Abziehen des Lösungsmittels wurde die Base in kristalliner Form erhalten.

Zur Durchführung der Versuche wurden 5 - 7 Wochen alten haarlosen Mäusen Hautabschnitte entnommen. Diese wurden so in übliche Diffusionszellen eingespannt, daß die Hornhautschicht auf der Seite der Donorflüssigkeit lag.

Zur Entfernung abgestorbener Schuppen wurde die Hornhaut zwei Stunden physiologischer Kochsalzlösung ausgesetzt. Danach wurde diese durch eine Diltiazem-haltige Lösung ersetzt. Die Empfänger-Lösung bestand bei Formulierungen mit niedrigem Flux aus physiologischer Kochsalzlösung für solche mit höherem Flux aus isotonem Citratpuffer (pH, 4,5).

Die Empfängerlösung wurde nach Entnahme eines bestimmten Volumens der Flüssigkeit in bestimmten Zeitintervallen auf deren Gehalt an Diltiazem untersucht. Die entnommene Flüssigkeitsmenge wurde durch frische Lösung ersetzt. Vor der Gehaltsbestimmung im Hochdruckflüssigkeitschromatographen wurde die Lösung durch einen $0,45\mu$ Nylonfilter gefiltert. Die Versuchstemperatur betrug 37°C.

Es kann davon ausgegangen werden, daß der Flux bei menschlicher Haut etwa 1/5 des Flux von Mäusehaut beträgt, so daß die ermittelten Fluxwerte mit dem Faktor 0,2 versehen werden müssen, wenn man auf die Permeabilität menschlicher Haut schließen will.

Die folgende Tabelle 1 enthält die aus verschiedenen Lösungsmittelsystemen ermittelten Fluxwerte und Permeabilitätswerte. Die Fluxwerte geben an, welche Menge unter den angebenenen Versuchsbedingungen über eine Hautfläche von 1 cm$^2$ während einer Stunde von der Donor zur Akzeptorseite diffundiert. Die Totzeit (Lag Time) ist die Zeit von Versuchsbeginn bis zur Gewinnung erster positiver Analysenwerte.

Es wurden folgende Lösungsmittel untersucht:

1.) Octanol (OC)

2.) Propylenglycol (PG)

3.) Oleylalkohol (OA)

4.) Linolsäure (LA)

5.) Triacetin (TA)

Die angegebenen Verhältnisse beziehen sich auf das Gewicht.

TABELLE I

| Lösungsmittel | Donor-Konzentration (mg/ml) | Flux ($\mu$g/cm$^2$/h) | Totzeit (h) | Permeabilität (cm/s x 10$^7$) |
|---|---|---|---|---|
| OC : PG 20 : 80 | 52.42 | 149.27 | 5.2 | 7.9 |
| OA : PG 20 : 80 | 45.75 | 83.71 | 0.82 | 5.1 |
| LA : PG 2,5:97,5 | 62.84 | 610.5 | 15.4 | 27.0 |
| LA : PG 5 : 95 | 75.37 | 555.09 | 14.5 | 20.5 |
| LA : PG 10 : 90 | 83.24 | 687.64 | 12.5 | 22.9 |
| LA : PG 20 : 80 (Lösung) | 126.46 | 165.32 | 4.0 | 3.6 |
| LA : PG : TA 20 : 30 : 50 | 90.64 | 231.54 | 15.2 | 7.1 |

Aus der Tabelle I ist ersichtlich, daß LA:PG Mischungen mit einem Überschuß PG besonders hohe Fluxwerte ergeben.

EP 0 214 620 B1

Bei dem besten Flux-Wert (LA:PG, 10:90) von 687,64 würde innerhalb von 24 Stunden durch eine Fläche von 30 cm$^2$ menschlicher Haut folgende Menge Wirkstoff aufgenommen:

$$\frac{687,64 \times 30 \times 24}{1000 \times 5} = \underline{99,02 \text{ mg}}$$

Unter Berücksichtigung des fehlenden First-Pass-Effekts entspräche dies einer oralen Dosis von ca. 180 - 200 mg Diltiazem.

Die folgenden Beispiele dienen zur näheren Erläuterung der Erfindung:

**Beispiel 1**

Aus 0,5 g Diltiazem-Base, 2,0 ml Isopropylmyristat und 10,0 ml Ethanol wird eine Lösung bereitet von der 2 ml über ein ganzes Schweineohr (Außenfläche) eines Schweines von 40 kg verteilt werden. Bereits nach 25 Minuten ist ein Diltiazem Plasmaspiegel von 90 ng/ml feststellbar, der nach 60 Minuten auf 30 ng/ml absinkt.

**Beispiel 2**

10 g Linolsäure werden mit 90 g Propylenglycol gemischt. In dieser Mischung löst man 8,324 g Diltiazem-Base und tränkt mit dieser Lösung einseitig mit Kunststoff beschichtete Gaze-Quadrate von 30 cm$^2$ Fläche (entspr. 5,5 cm Kantenlänge). Die Quadrate werden dann in Aluminiumfolie eingesiegelt.

**Patentansprüche**

1. Transdermale Applikationsform für Menschen und Säugetiere, enthaltend Diltiazem-Base als Wirkstoff, der, gegebenenfalls mit an sich bekannten Hilfs- und Zusatzstoffen, in einem hautverträglichen Lösungsmittel gelöst oder suspendiert ist.

2. Transdermale Applikationsform nach Anspruch 1, dadurch gekennzeichnet, daß diese als Lösungsmittel ein Gemisch von Linolsäure (LA) und Propylenglykol (PG) enthält.

3. Transdermale Applikationsform nach Anspruch 2, dadurch gekennzeichnet, daß diese Linolsäure (LA) und Propylenglykol (PG) in einem Gewichtsverhältnis von 1 - 20 Gew.-% LA und 99 - 80 Gew-.% PG enthält.

4. Transdermale Applikationsform nach Anspruch 1 bis 3, dadurch gekennzeichnet, daß diese aus Mull- oder Gazequadraten von 3-10 cm Kantenlänge besteht, welche mit einer Lösung von Diltiazem-Base in einem Gemisch von 10 Gew-.% Linolsäure und 90 Gew-.% Propylenglykol imprägniert sind.

5. Verfahren zur Herstellung einer transdermalen Applikationsform eines Wirkstoffs für Menschen und Säugetiere, dadurch gekennzeichnet, daß der Wirkstoff Diltiazem-Base ist, welcher in einem hautverträglichen Lösungsmittel, gegebenenfalls mit weiteren Hilfs- und Zusatzstoffen, gelöst oder suspendiert wird.

6. Verfahren nach Anspruch 5, dadurch gekennzeichnet, daß der Wirkstoff Diltiazem-Base und das Lösungsmittel Ethanol, Methylpyrrolidon, Polyethylenglykol, Oleylalkohol, Octanol, Linolsäure, Triacetin, Propylenglykol, Glycerin, Solketal oder Dimethylsulfoxid ist.

7. Verfahren nach Anspruch 6, dadurch gekennzeichnet, daß das Lösungsmittel aus einer Mischung von 1 - 20 Gew-.% Linolsäure (LA) und 99 - 80 Gew-.% Propylenglykol besteht.

8. Verfahren nach Anspruch 4 - 7, dadurch gekennzeichnet, daß mit dem im Lösungsmittel gelösten oder suspendierten Wirkstoff ein saugfähiger Träger bestimmter Oberfläche getränkt wird.

EP 0 214 620 B1

**9.** Verwendung von Diltiazem-Base für die Herstellung transdermaler Applikationsformen für Menschen und Säugetiere.

**10.** Verwendung von Diltiazem-Base nach Anspruch 9 für die Herstellung von transdermalen Arzneimitteln zur Verminderung der Streßbelastung von Schweinen.

**Claims**

**1.** A transdermal form of application for humans and mammals, containing diltiazem base as an active substance, which is dissolved or suspended in a solvent with good skin compatibility, possibly with auxiliary agents and admixed materials known per se.

**2.** A transdermal form of application according to claim 1, characterised in that this contains as solvent a mixture of linolic acid (LA) and propylene glycol (PG).

**3.** A transdermal form of application according to claim 2, characterised in that this contains linolic acid (LA) and propylene glycol (PG) in a weight ratio of 1 - 20 % by weight LA and 99 - 80 % by weight PG.

**4.** A transdermal form of application according to claim 1 to 3, characterised in that this consists of fine muslin or gauze squares of 3-10 cm edge length, which are impregnated with a solution of diltiazem base in a mixture of 10 % by weight linolic acid and 90% by weight propylene glycol.

**5.** A method for manufacturing a transdermal form of application of an active substance for humans and mammals, characterised in that the active substance is diltiazem base, which is dissolved or suspended in a solvent with good skin compatibility, possibly with further auxiliary agents and admixed materials.

**6.** A method according to claim 5, characterised in that the active substance is diltiazem base and the solvent ethanol, methyl pyrollidone, polyethylene glycol, oleyl alcohol, octanol, linolic acid, triacetine, propylene glycol, glycerin, solketal or dimethyl sulphoxide.

**7.** A method according to claim 6, characterised in that the solvent consists of a mixture of 1 - 20 % by weight linolic acid (LA) and 99 - 80 % by weight propylene glycol.

**8.** A method according to claim 4 - 7, characterised in that an absorbent carrier of a particular surface is impregnated with the active substance dissolved or suspended in the solvent.

**9.** A use of diltiazem base for the manufacture of transdermal forms of application for humans and mammals.

**10.** A use of diltiazem base according to claim 9 for the manufacture of transdermal drugs for reducing the stress of pigs.

**Revendications**

**1.** Forme d'application transdermique destinée à des êtres humains et à des mammifères contenant du diltiazem-base comme substance active que, le cas échéant, on met en solution ou en suspension dans un solvant compatible pour la peau accompagné le cas échéant d'auxiliaires et d'additifs connus.

**2.** Forme d'application transdermique selon la revendication 1, caractérisée en ce que celle-ci contient comme solvant un mélange d'acide linoléique (LA) et de propylène glycol (PG).

**3.** Forme d'application transdermique selon la revendication 2, caractérisée en ce qu'elle contient de l'acide linoléique (LA) et du propylène glycol (PG) dans un rapport pondéral de 1 à 20 % en poids de LA et 99 à 80 % en poids de PG.

6

**4.** Forme d'application transdermique selon l'une quelconque des revendications 1 à 3, caractérisée en ce qu'elle est constituée de carrés de mousseline ou de gaze de 3 à 10 cm de longueur de côté qui sont imprégnées d'une solution de diltiazem-base dans un mélange formé de 10 % en poids d'acide linoléique et 90 % en poids de propylène glycol.

**5.** Procédé de préparation d'une forme d'application transdermique d'une substance active destinée à des êtres humains et à des mammifères, caractérisé en ce que la substance active est le diltiazem-base que l'on met en solution ou en suspension dans un solvant compatible avec la peau accompagné le cas échéant d'autres auxiliaires ou additifs.

**6.** Procédé selon la revendication 5, caractérisé en ce que la substance active est le diltiazem-base et le solvant l'éthanol, la méthylpyrrolydone, le polyéthylène glycol, l'alcool oléique, l'octanol, l'acide linoléique, la triacétine, le propylène glycol, la glycérine, le solcétal ou le diméthylsulfoxyde.

**7.** Procédé selon la revendication 6, caractérisé en ce que le solvant est constitué d'un mélange de 1 à 20 % d'acide linoléique (LA) et 99 à 80 % en poids de propylène glycol (PG).

**8.** Procédé selon l'une quelconque des revendications 4 à 7, caractérisé en ce que l'on imprègne un support absorbant présentant une certaine surface à l'aide de la solution ou suspension de subtance active dans ledit solvant.

**9.** Utilisation de diltiazem-base pour la préparation de formes d'application transdermique destinées aux êtres humains et aux mammifères.

**10.** Diltiazem-base selon la revendication 9, pour la préparation de médicaments transdermiques destinés à diminuer la charge de stress chez le porc.